# EUROPEAN PATENT APPLICATION

(11) **EP 1 873 150 A1**
(43) Date of publication of application: **02.01.2008**
(21) Application number: 06116414.1
(22) Date of filing: 30.06.2006
(51) Int. Cl.: C07D 401/06, C07D 401/14, C07D 491/08, A61K 31/454, A61K 31/439, A61P 29/00

(54) **Fluorinated indoleamides useful as ligands of the ORL-1 receptor**

(71) Applicant: NIKEM RESEARCH S.R.L., 20021 Baranzate di Bollate Milano (IT)
(72) Inventor: Ronzoni, Silvano, 20021 Baranzate di Bollate (IT); Gagliardi, Stefania, 20021Baranzate di Bollate (IT)
(74) Representative: Gervasi, Gemma

(57) **Abstract**

New ligands for the ORL-1 receptor are described, useful for antagonising the activity of said receptors in a patient in need thereof, and for preventing and treating illnesses dependent on the activation of this receptor. The new compounds conform to structural formula (I)

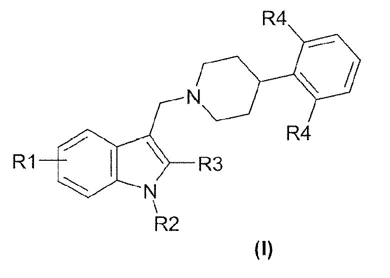

In formula (I) *inter alia,* at least one of the two R₄ substituents is a fluorine atom and at least one among R2 and R3 is a group -(CH₂)ₙ-CONRₐR_{b}, where R1, R2, R3, R4, n, Rₐ, R_{b} are defined in the description.

## Description

### STATE OF THE ART

The present invention relates to certain new compounds, a process for their preparation, to pharmaceutical compositions which contain them, and to the use of these compounds in medicine. The invention relates in particular to a group of new compounds possessing antagonistic activity for the receptors ORL-1 and useful in treating illness dependent on the activation of these receptors.

The ORL-1 receptor is located along the entire neural axis and is involved in various pathological phenomena, including the transmission of pain. Various peptide and non-peptide ligands for the ORL-1 receptor are known; the non-peptide ligands include known compounds with morphinan, benzimidazopiperidine, spiropiperidine, arylpiperidine and 4-aminoquinoline structure *(*Life Sciences, 73, 2003, 663-678). WO 0183454 and WO 03040099 describe other ORL-1 antagonists with benzosuberonylpiperidine structure substituted in position 5 by a hydroxy, alkoxy, amino or alkylamino group, and their synthesis method. J.Med.Chem., 1997, 40(23), 3912-14 and WO 9709308 describe certain indoles substituted in position 3 with a dipiperazine group, as antagonists for the receptor NPY-1. J.Med.Chem., 1996, 39(10), 1941-2, WO 9424105, WO 9410145, WO 02241894, WO 9629330 and GB 2076810 describe variously substituted 3-piperazinylmethyl indoles as ligands for dopamine receptors, in particular for the D4 receptor. GB 2083476 describes specific 3-arylpiperidinylmethyl indoles as 5HT uptake inhibitors. US 5215989 describes certain di-substituted piperazine and imidazole derivatives useful as class III antiarrhythmic agents. EP 846683 describes hydroxypiperidine derivatives as NMDA receptor blockers. WO 200241894 describes 2-piperazino substituted indoles, useful as antagonists for the dopamine D4 receptors. GB 1063019 describes certain piperidinoalkyl substituted indoles, useful as myorelaxants.

Patent application PCT/EP2005/057192 describes ORL-1 receptor ligands of indole amide structure.

Patent application WO2005005411 describes a large family of compounds of formula

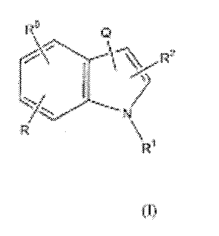

They are described as ORL-1 ligands, although no affinity data are reported.

A problem often present for many pharmaceutical substances, including the ligands of the above formula (I), is represented by a significant inhibitory activity towards the cytochrome P450 isoenzyme family, which is the main responsible for the metabolism of xenobiotics. As a consequence, these substances, when administered in combined therapy with other drugs, may cause a high risk of undesired drug-drug interactions due to altered metabolism of the coadministered drug.

In view of the above prior art the need is felt for new highly active ORL-1 ligands.

The need is felt, in particular, for ORL-1 ligands having a high binding affinity to the ORL-1 receptor and, at the same time, a low or absent inhibitory effect on P450 cytochrome.

### SUMMARY OF THE INVENTION

The present invention refers to a new familly of substituted indoles being powerful ligands for the ORL-1 receptor, and thus useful in the treatment and/or prevention of diseases dependent on the activation of this receptor.

They can thus be used in man or animals for treating and/or preventing pain, gastrointestinal disorders, diseases of the immune system, dysfunctions of the cardiovascular system, diseases of the excretory system, sexual dysfunction, disorders of the respiratory tract, central nervous system disorders, drug abuse, tolerance and dependence, etc. Examples of specific diseases dependent on the activation of the ORL-1 receptor are listed further on in this specification.

The compounds of the invention are conform to structural formula (I) 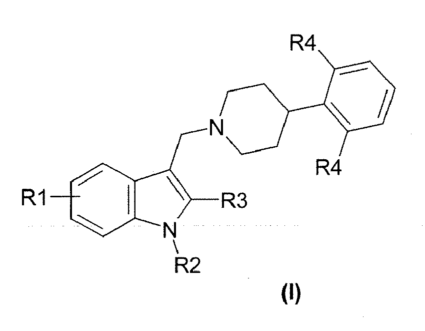 wherein:
**R1** is hydrogen; halogen; C₁₋₆alkyl; perhaloC₁₋₆alkyl; aryl;
**R2** and **R3** are, independently from each other:
   hydrogen; C₁₋₆alkyl, aryl; arylC₁₋₆alkyl; -(CH₂)ₙCONRₐR_{b} where n = 0-4; Rₐ and R_{b} are, independently from each other: hydrogen; C₁₋₆alkyl; C₃₋₆cycloalkyl; aryl; arylC₁₋₆alkyl; heteroarylC₁₋₆alkyl; saturated or unsaturated, optionally substituted heterocycle containing 1 to 3 heteroatoms chosen from O, S, N; or Rₐ and R_{b} together with the nitrogen atom to which they are attached, form a saturated or unsaturated, optionally substituted heterocycle containing a total of 1 to 3 heteroatoms chosen from O, S, N, and whose ring members may be bound with each other by a C₁₋₄ alkyl bridge, or said heterocycle may be spiro-substituted at any position by an optionally substituted nitrogen-containing heterocycle, and with the proviso that at least one among R2 and R3 is -(CH₂)ₙCONRₐR_{b};
the **R4** groups are, independently from each other, hydrogen or halogen, with the proviso that at least one R4 group is a fluorine atom.

### DETAILED DESCRIPTION OF THE INVENTION

In the present invention, all "aryl" groups, including those where the aryl is part of larger groups, e.g. arylC₁₋₆alkyl, are preferably C₅₋₁₀aryl groups, in particular phenyl and naphthyl; said aryl may be substituted one or more times by e.g. halogen, C₁₋₆alkoxy, C₁₋₆alkyl, hydroxy, amino, C₁₋₆alkylamino, di(C₁₋₆alkyl)amino, aminoC₁₋₆alkyl, (C₁₋₆alkyl)aminoC₁₋₆alkyl, di(C₁₋₆alkyl)aminoC₁₋₆alkyl, aryl, cyano or perhaloC₁₋₆alkyl.

In the present invention, all "alkyl" groups, including those where the alkyl is part of larger groups, e.g. arylC₁₋₆alkyl, can be linear or branched; said alkyl groups are preferably C₁₋₂alkyl groups, more preferably methyl.

The term "halogen" includes the iodine, chlorine, bromine and fluorine groups, preferably chlorine, fluorine and bromine.

The term "spiro-substituted" means that the two involved rings are interconnected with each other by sharing one ring member.

Whenever ring members of said heterocycle are "bound with each other by a C₁₋₄ alkyl bridge", the resulting structure is a bicyclo-ring; an example thereof is the compound of Example 9.

Preferably, R1 is chosen among hydrogen or halogen; more preferably, R1 is hydrogen or fluoro.

In the aforesaid -(CH₂)ₙCONRₐR_{b} group, *n* is preferably 0 or 1; Rₐ and R_{b} are preferably hydrogen or an optionally substituted saturated heterocycle containing 1 to 3 heteroatom chosen from O, S, N, or Rₐ and R_{b} together with the nitrogen atom to which they are attached, form an optionally substituted saturated heterocycle containing 1 to 3 heteroatoms chosen from O, S, N, whose ring members may be bound with each other by a C₁₋₂ alkyl bridge.

Specific preferred moieties representative of the -(CH₂)ₙCONRₐR_{b} group are -CONH₂ , -CH₂-CONH₂, and the following ones: 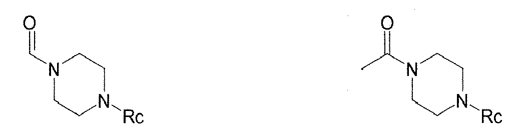 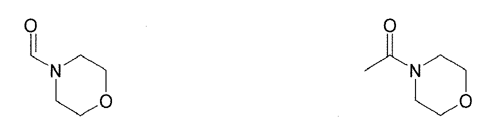 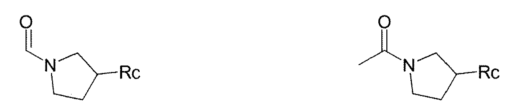 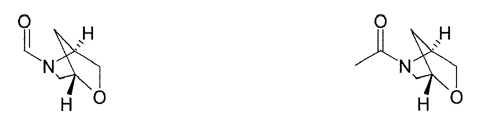 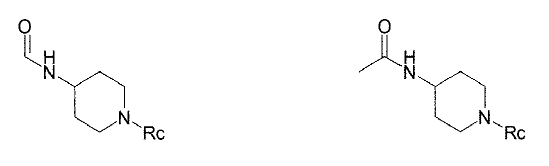 where R_{c} is C₁₋₆ linear or branched alkyl, C₁₋₆alkylCON(R_{d}) where R_{d} is H or C₁-₆ linear or branched alkyl, hydroxyC₁₋₆ alkyl wherein one member of said alkyl can be replaced by oxygen, heterocyclylcarbonyl or heterocyclylcarbonylC₁₋₆alkyl wherein said heterocyclyl is preferably piperidin-1-yl, morpholin-4-yl, piperazin-1-yl.

The R2 or R3 group different from -(CH₂)ₙCONRₐR_{b}, if present, is preferably chosen among hydrogen, C₁₋₆alkyl, phenyl.

The R4 substituent different from fluorine, if present, is preferably chlorine.

Specific compounds of formula (I) according to the present invention (of which each also comprises the corresponding salts such as hydrochloride or trifluoroacetate), and hydrates, are the following:
2-(4-{3-[4-(2-Chloro-6-fluoro-phenyl)-piperidin-1-ylmethyl]-1H-indole-2-carbonyl}-piperazin-1-yl)-1-morpholin-4-yl-ethanone
2-(4-{3-[4-(2,6-Difluoro-phenyl)-piperidin-1-ylmethyl]-1H-indole-2-carbonyl}-piperazin-1-yl)-1-morpholin-4-yl-ethanone
N-(1-{3-[4-(2-Chloro-6-fluoro-phenyl)-piperidin-1-ylmethyl]-1H-indole-2-carbonyl}-pyrrolidin-3-yl)-N-methyl-acetamide
N-(1-{3-[4-(2,6-Difluoro-phenyl)-piperidin-1-ylmethyl]-1H-indole-2-carbonyl}-pyrrolidin-3-yl)-N-methyl-acetamide
{3-[4-(2-Chloro-6-fluoro-phenyl)-piperidin-1-ylmethyl]-1H-indol-2-yl}-{4-[2-(2-hydroxy-ethoxy)-ethyl]-piperazin-1-yl}-methanone
{3-[4-(2,6-Difluoro-phenyl)-piperidin-1-ylmethyl]-1H-indol-2-yl}-{4-[2-(2-hydroxy-ethoxy)-ethyl]-piperazin-1-yl}-methanone
2-[3-[4-(2-Chloro-6-fluoro-phenyl)-piperidin-1-ylmethyl]-2-(morpholine-4-carbonyl)-indol-1-yl]-acetamide
2-[3-[4-(2,6-Difluoro-phenyl)-piperidin-1-ylmethyl]-2-(morpholine-4-carbonyl)-indol-1-yl]-acetamide
{3-[4-(2-Chloro-6-fluoro-phenyl)-piperidin-1-ylmethyl]-1H-indol-2-yl}-(1S,4S)-2-oxa-5-aza-bicyclo[2.2.1]hept-5-yl-methanone
{3-[4-(2,6-Difluoro-phenyl)-piperidin-1-ylmethyl]-1H-indol-2-yl}-(1S,4S)-2-oxa-5-aza-bicyclo[2.2.1]hept-5-yl-methanone hydrochloride
2-{3-[4-(2-Chloro-6-fluoro-phenyl)-piperidin-1-ylmethyl]-2-methyl-indol-1-yl}-N-(1-methyl-piperidin-4-yl)-acetamide
2-{3-[4-(2-Chloro-6-fluoro-phenyl)-piperidin-1-ylmethyl]-2-phenyl-indol-1-yl}-1-morpholin-4-yl-ethanone
2-{3-[4-(2,6-Difluoro-phenyl)-piperidin-1-ylmethyl]-2-phenyl-indol-1-yl}-1-morpholin-4-yl-ethanone
2-{3-[4-(2-Chloro-6-fluoro-phenyl)-piperidin-1-ylmethyl]-2-phenyl-indol-1-yl}-1-(4-methyl-piperazin-1-yl)-ethanone
2-{3-[4-(2,6-Difluoro-phenyl)-piperidin-1-ylmethyl]-2-phenyl-indol-1-yl}-1-(4-methyl-piperazin-1-yl)-ethanone
2-{3-[4-(2,6-Difluoro-phenyl)-piperidin-1-ylmethyl]-2-methyl-indol-1-yl}-1-(4-methyl-piperazin-1-yl)-ethanone

The compounds of formula (I) can exhibit stereoisomerism because of the presence of chiral atoms and/or multiple bonds. The present invention therefore extends to stereoisomers of the compounds of the formula (I), including racemes, enantiomers, diastereoisomers and geometric isomers.

It has been found that, when a compound of formula (I) exhibits optical isomerism, an enantiomer possesses a greater affinity for the ORL-1 receptor than its antipod.

Consequently, the present invention also provides an enantiomer of a compound of formula (I) as above defined.

In a further aspect, the present invention provides a mixture of enantiomers of a compound of formula (I) as above defined, where an enantiomer is present in a proportion greater than its antipod.

The subject invention also includes isotopically-labelled compounds, which are identical to those recited in formula (I) and following, but for the fact that one or more atoms are replaced by an atom having an atomic mass or mass number different from the atomic mass or mass number usually found in nature.

Examples of isotopes that can be incorporated into compounds of the invention and pharmaceutically acceptable salts thereof include isotopes of hydrogen, carbon, nitrogen, oxygen, phosphorous, sulphur, fluorine, iodine, and chlorine, such as ²H, ³H, ¹¹C, ¹³C, ¹⁴C, ¹⁵N, ¹⁷O, ¹⁸O, ³¹P, ³²P, ³⁵S, ¹⁸F, ³⁶Cl, ¹²³I and ¹²⁵I.

Compounds of the present invention and pharmaceutically acceptable salts of said compounds that contain the aforementioned isotopes and/or other isotopes of other atoms are within the scope of the present invention. Isotopically-labelled compounds of the present invention, for example those into which radioactive isotopes such as ³H, ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. ¹¹C and ¹⁸F isotopes are particularly useful in PET (positron emission tomography), and ¹²⁵I isotopes are particularly useful in SPECT (single photon emission computerized tomography), all useful in brain imaging. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labelled compounds of formula I and following of this invention can generally be prepared by carrying out the procedures disclosed in the Schemes and/or in the Examples below, by substituting a readily available isotopically labelled reagent for a non-isotopically labelled reagent.

The present invention also provides processes for preparing the compounds of formula (I).

Compounds of formula (I) in which R2 is hydrogen, hereinafter referred as formula (Ia), can be obtained reacting a compound of formula (II), 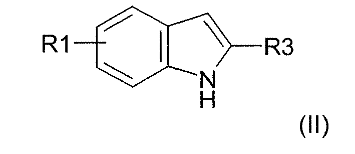 in which R1 and R3 have the meanings described for formula (I), with formaldehyde and a compound of formula (III), 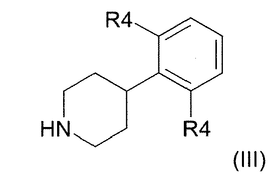 in which R4 has the meanings described for formula (I).

This reaction is typically a Mannich reaction, as described in standard reference texts of synthetic methodology, such as J. March, Advanced Organic Chemistry, 3rd Edition (1985), Wiley Interscience.

In particular, compounds of formula (Ia) can be prepared in accordance with scheme 1, starting from compounds of formula (II), formaldehyde and an amine of formula (III).

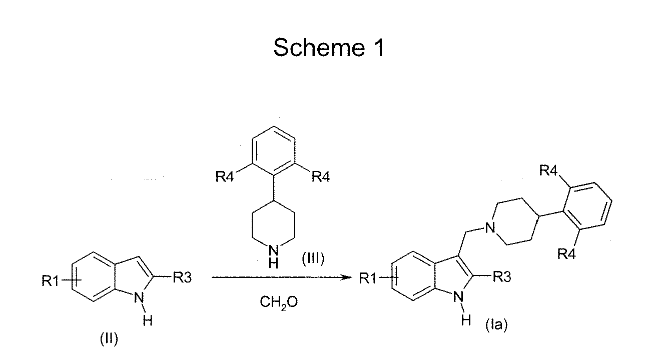

In a typical procedure, an amine of formula (III) is dissolved in a suitable solvent, such as for example methanol or dioxane or a mixture of both, to which solution an aqueous solution of formaldehyde and acetic acid are added. After a suitable time, typically between 5 min and 1 h, there is added to the preceding reaction mixture a solution of an indole of formula (II) in a suitable solvent, such as for example methanol or dioxane or a mixture of both, while maintaining the temperature of the resultant solution generally between 0°C and ambient temperature. The reaction mixture is stirred for a suitable time, typically between 1 h and 96 h, at a suitable temperature, typically between 0°C and 80°C, after which it is processed by known methods.

Two preferred processing procedures are here indicated as procedure A and procedure B.

In procedure A, water is added to the reaction mixture followed by a solution of a suitable base, such as aqueous ammonium hydroxide or sodium hydroxide, until basic pH is reached, after which it is extracted with a suitable organic solvent such as ethyl acetate. The organic phase is collected and dried with, for example, sodium sulfate, and the solvent is removed by evaporation. The crude product can be purified, if necessary, by conventional purification methods such as flash chromatography, trituration, crystallization or preparative HPLC.

In procedure B, the reaction mixture is poured onto an acid resin cartridge and eluted with a suitable solvent, such as for example dichloromethane or methanol, to remove non-basic impurities, and then with a solution of a suitable base in a suitable organic solvent such as, for example, a methanolic ammonia solution, to recover the desired compound of formula (I). The solvent is removed by evaporation and the crude product can be purified, if necessary, by conventional purification methods such as flash chromatography, trituration, crystallization or preparative HPLC.

Compounds of formula (II) are known or commercially available compounds or can be prepared by procedures described in standard reference texts of synthesis methodologies such as J. March, Advanced Organic Chemistry, 3rd Edition (1985), Wiley Interscience.

The compounds of formula (III) can be prepared e.g. by the procedures described in WO 01/83454.

Compounds of formula (I) in which R2 is different from hydrogen, hereinafter referred as formula (Ib), can be prepared as follows: a compound of formula (II) is treated in accordance with the two following steps, which can take place in any order:
a) reaction with formaldehyde and a compound of formula (III)
b) reaction with a compound of formula R2-W, in which R2 is as defined in formula (I) and W is a suitable leaving group,
thus obtaining the compounds of formula (Ib).

If the steps are carried out in the order (a) → (b), the compound of formula (II) is firstly functionalized in position 3 by reaction with formaldehyde and the compound of formula (III); the 3-functionalized intermediate obtained is then N-alkylated in position 1 of the indole ring by treatment with the compound R2-W, to obtain the final compound of formula (Ib).

If the steps are carried out in the reverse order (b) → (a), the compound of formula (II) is firstly N-alkylated in position 1 of the indole ring by reaction with the compound R2-W; the N-alkylated intermediate obtained is then 3-functionalized by reaction with formaldehyde and the compound of formula (III), to obtain the final compound of formula (Ib).

Step (a) is effected preferably by the Mannich reaction, in the previously detailed manner.

Step (b) is a nucleophilic reaction which can be effected by commonly known methods; in particular it is effected by reacting the compound of formula (II) (or, as illustrated in the following Scheme 2, its 3-substituted derivative resulting from step (a)) with a strong base and then treating the resultant indolyl anion with said compound of formula R2-W.

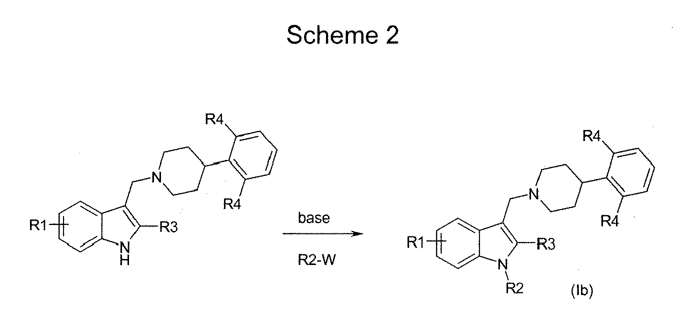

In a typical procedure, a suitable base such as sodium hydride, is added under an inert atmosphere, typically of argon or nitrogen, to a solution of a compound of formula (II) or its 3-substituted derivative in a suitable anhydrous solvent, such as dimethylformamide, at a suitable temperature, typically between 0°C and ambient temperature. After a suitable time, typically between 15 min and 1 h, a suitable alkylating agent of formula R2-W is added to the reaction mixture, either as such or dissolved in a suitable anhydrous solvent such as dimethylformamide; if necessary, further additions of alkylating agent can be made. The resultant reaction mixture is stirred at a suitable temperature, typically ambient temperature, for a suitable time, typically between 1 h and 20 h. The procedure can be carried out by known methods. Two preferred working procedures are here indicated as procedure A and procedure B.

In procedure A, water is added to the reaction mixture, which is then extracted with a suitable organic solvent such as diethylether. The organic phase is collected and dried with, for example, sodium sulfate, and the solvent is removed by evaporation. The crude product can be purified, if necessary, by conventional purification methods such as flash chromatography, trituration, crystallization and preparative HPLC.

In working procedure B, water is added to the reaction mixture, which is then filtered through a suitable water retention filter, eluting with a suitable solvent such as ethyl acetate. The resultant solution can be concentrated, if necessary, and then poured onto an acid resin cartridge and eluted with a suitable solvent, such as methanol, to remove non-basic impurities, and then with a solution of a suitable base in a suitable organic solvent such as a methanolic solution of ammonia, to recover the desired compound of formula (I). The solvent is removed by evaporation and the crude product can be purified, if necessary, by conventional purification methods such as flash chromatography, trituration, crystallization and preparative HPLC.

Compounds of formula (I) in which R3 is a group -(CH₂)ₙCONRₐR_{b} and n = 0, hereinafter referred as formula (Ic), can be prepared in accordance with scheme 3, activating an amine of formula HNRₐR_{b} with trimethylaluminium and then reacting said activated amine with a compound of formula (IV), 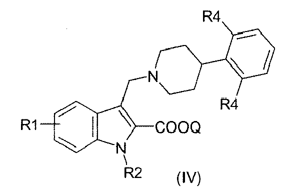 where R1, R2 and R4 have the meanings given for formula (I), and Q is a linear or branched C₁₋₄alkyl, under conditions reported for example in Basha et al., Tetrahedron Lett., 18, 4171, (1977).

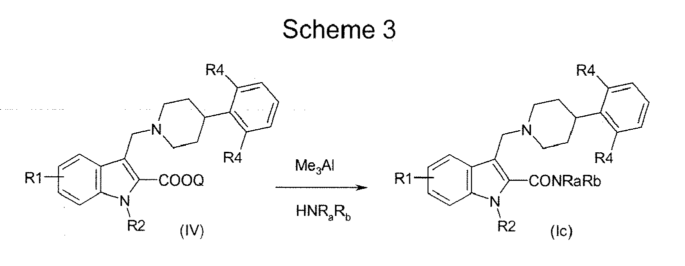

In a typical procedure, a solution of trimethylaluminium in a suitable solvent, such as for example hexane or toluene, is added at a suitable temperature, typically between 0°C and room temperature, to a solution of an amine of formula HNRₐR_{b} dissolved in a suitable anhydrous solvent, such as for example dichloromethane or toluene, under an inert atmosphere, typically of nitrogen or argon. After a suitable time, typically between 5 min and 1 h, there is added to the preceding reaction mixture a solution of a compound of formula (IV) dissolved in a suitable anhydrous solvent, such as for example dichloromethane or toluene, while maintaining the temperature of the resultant solution generally between 0°C and room temperature. The reaction mixture is stirred for a suitable time, typically between 30 min to 48 h, at a suitable temperature, typically between room temperature and 110°C, after which water is added and the reaction mixture is extracted with a suitable solvent such as ethyl acetate or dichloromethane. The organic phase is collected and dried with, for example, sodium sulfate and the solvent is removed by evaporation. The crude product can be purified, if necessary, by conventional purification methods such as flash chromatography, trituration, crystallization and preparative HPLC.

Alternatively, compounds of general formula (Ic) can be prepared as described in scheme 4, reacting a suitably activated carboxylic acid of formula (V) with an amine of formula HNRₐR_{b}. 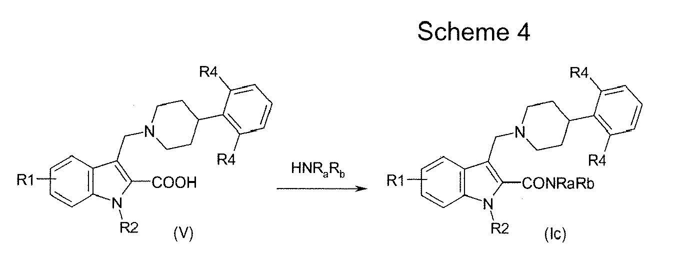 where R1, R2 R4 and X have the meanings given for formula (I).

Activation of the compound of formula (V), effected before reacting with the compounds of formula HNRₐR_{b} can suitably take place by forming the corresponding acyl halides, for example by reaction with oxalyl chloride or thionyl chloride; alternatively, the compounds of formula (V) can also be activated using activating agents such as 1,1'-carbonyldiimidazole, dicyclohexylcarbodiimide/1-hydroxybenzotriazole, N-ethyl-N'-(3-dimethylamino-propyl)carbodiimide/1-hydroxybenzotriazole, O-benzotriazol-1-yl-N,N,N',N'-tetramethyluronium hexafluorophosphate or (benzotriazol-1-yloxy)tripyrrolidinophosphonium hexafluorophosphate.

In a typical procedure, a solution of a compound of formula (V) in a suitable solvent, such as for example dimethylformamide or acetonitrile, is treated with a suitable activating agent, such as for example 1,1'-carbonyldiimidazole or (benzotriazol-1-yloxy)tripyrrolidinophosphonium hexafluorophosphate, either with or without a suitable base such as, for example, triethylamine or N-ethyldiisopropylamine, at a suitable temperature, typically between 0°C and room temperature. After a suitable period of time, typically between 30 min and 6 h, there is added to the preceding reaction mixture an amine of formula, HNRₐR_{b} either neat or dissolved in a suitable solvent, such as for example dimethylformamide or acetonitrile. After stirring for a suitable period of time, typically between 1 h and 24 h, the solvent is removed by evaporation and the residue is taken up in a suitable solvent, such as for example ethyl acetate or dichloromethane, washed with water or if necessary with a suitable basic solution, such as for example a saturated sodium bicarbonate solution. The organic phase is collected and dried with, for example, sodium sulfate and the solvent is removed by evaporation. The crude product can be purified, if necessary, by conventional purification methods such as flash chromatography, trituration, crystallization and preparative HPLC.

Alternatively, a compound of formula (V) is dissolved in thionyl chloride and the resulting reaction mixture is stirred at a suitable temperature, typically between room temperature and 60°C, for a suitable period of time, typically between 1 h and 8 h. The excess thionyl chloride is removed by evaporation and the resulting acyl chloride is dissolved in a suitable solvent, such as dimethylformamide or dichloromethane, and added at a suitable temperature, typically between 0°C and room temperature, to a solution of an amine of formula HNRₐR_{b} in a suitable solvent, such as dimethylformamide or dichloromethane. The reaction mixture is stirred for a suitable period of time, generally between 1 h and 16 h, then water is added, followed if necessary by a suitable basic solution, such as diluted sodium hydroxide solution, then the organic phase is collected, dried with, for example, sodium sulfate and the solvent is removed by evaporation. The crude product can be purified, if necessary, by conventional purification methods such as flash chromatography, trituration, crystallization and preparative HPLC.

Amines of formula HNRₐR_{b} are known or commercially available compounds or can be prepared by procedures described in standard reference texts of synthesis methodologies such as *J. March, Advanced Organic Chemistry, 3rd Edition (1985), Wiley Interscience.*

Compounds of formula (IV) or formula (V) can be prepared as described in scheme 5, starting from compounds of formula (VI) or formula (VII) respectively, an amine of formula (III) and formaldehyde, under the experimental conditions detailed above for the synthesis of compounds of formula (Ia).

Alternatively, compounds of formula (V) can be obtained from compounds of formula (IV) by hydrolysis of the group COOQ to the group COOH, under suitable basic (e.g. aqueous or alcoholic sodium or potassium hydroxide solution) or acidic (e.g. trifluoroacetic acid) conditions.

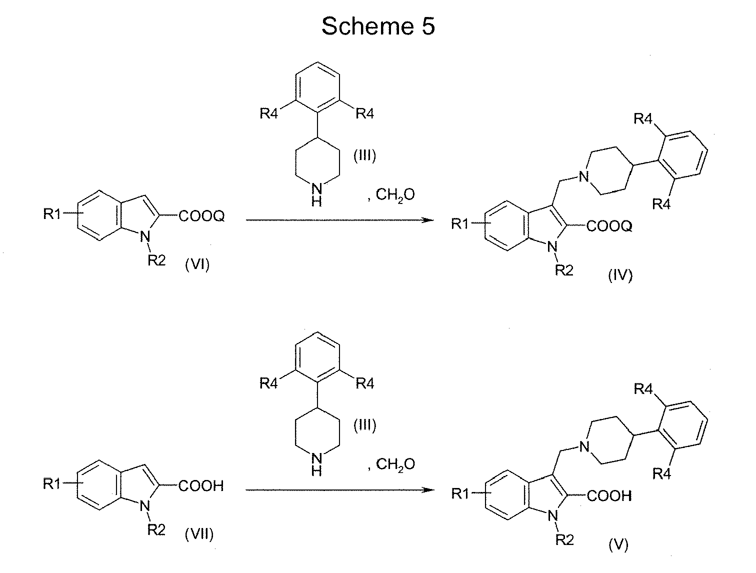

Compounds of formula (VI) and of formula (VII) are known or commercially available compounds or can be prepared by procedures described in standard reference texts of synthesis methodologies such as J. March, Advanced Organic Chemistry, 3rd Edition (1985), Wiley Interscience*.*

Compounds of formula (I) in which R2 is (CH₂)ₙCONRₐR_{b} and n = 1-4, hereinafter referred as formula (Id), can be prepared in accordance with scheme 6, reacting a compound of formula (Ia), with a compound of formula W-(CH₂)ₙCOOQ, where W is a suitable leaving group as described above, n = 1-4 and Q is as defined above, under alkylating conditions described above, to give compounds of formula (VIII), hydrolysing the ester group to the corresponding carboxylic acid of formula (IX) and then reacting a suitably activated form of acid (IX) with an amine of formula HNRₐR_{b}, where Rₐ and R_{b} have the meanings described for formula (I).

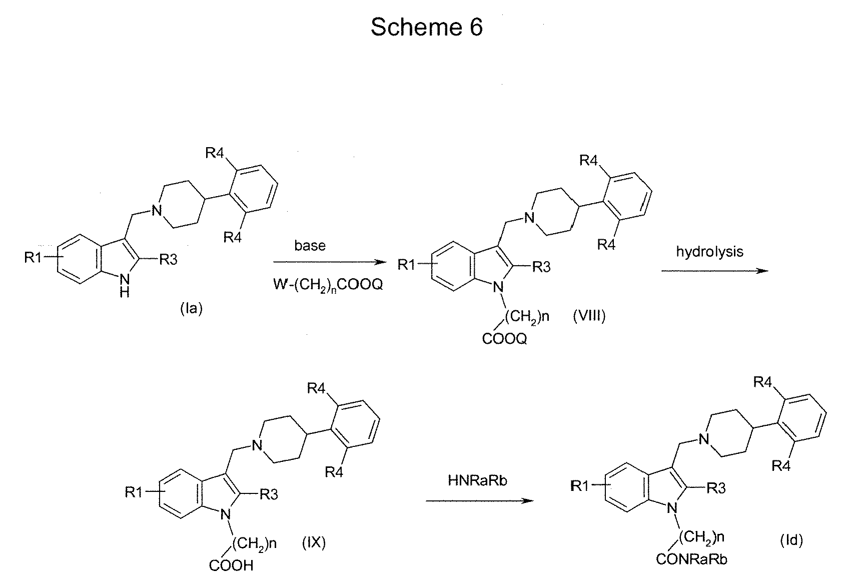

Hydrolysis of compounds of formula (VIII) can take place under basic (for example, sodium or potassium hydroxide solution) or acidic (for example, trifluoroacetic acid) conditions.

Activation of the compound of formula (IX), effected before reacting with the compounds of formula HNRₐR_{b}, can suitably take place by forming the corresponding acyl halides, for example by reaction with oxalyl chloride or thionyl chloride; alternatively, the compounds of formula (IX) can also be activated using activating agents such as 1,1'-carbonyldiimidazole, dicyclohexylcarbodiimide/1-hydroxybenzotriazole, N-ethyl-N'-(3-dimethylamino-propyl)carbodiimide/1-hydroxybenzotriazole, or O-benzotriazol-1-yl-N,N,N',N'-tetramethyluronium hexafluorophosphate.

In a typical procedure, trifluoroacetic acid is added to a solution of a compound of formula (VIII) in a suitable solvent, such as for example dichloromethane, at a suitable temperature, typically between 0°C and room temperature and the reaction mixture is stirred for a suitable time, typically between 1 and 24 h, after which the reaction mixture is evaporated to dryness. The crude compound of formula (IX) is dissolved in a suitable solvent, such as for example acetonitrile, and an activating agent such as 1,1'-carbonyldiimidazole is added to this solution and the reaction mixture is stirred for a suitable time, typically between 1 and 24 h, at a suitable temperature, typically between room temperature and 80°C, then an amine of general formula HNRaRb is added to the solution. The reaction mixture is stirred for a suitable time, generally between 15 min and 8 h, and at a suitable temperature, typically between room temperature and 80°C, then water is added and the reaction mixture is extracted with a suitable solvent, such as ethyl acetate or dichloromethane. The organic phase is collected and dried with, for example, sodium sulfate and the solvent is removed by evaporation. The crude product can be purified, if necessary, by conventional purification methods such as flash chromatography, trituration, crystallization and preparative HPLC.

Alternatively, compounds of general formula (Id) can be prepared in accordance with scheme 7, activating an amine of formula HNRₐR_{b}, where Rₐ and R_{b} have the meanings described for formula (I), with trimethylaluminium, and then reacting said activated amine with a compound of formula (VIII), under conditions reported above for the synthesis of compounds of general formula (Ic).

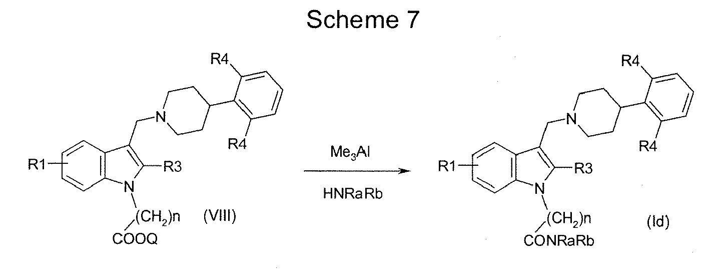

As aforestated, the compounds of formula (I) are antagonists of the ORL-1 receptor. Hence, a compound of formula (I) is provided as active therapeutic substance.

According to another aspect of the present invention a method is provided for antagonising the activity of the ORL-1 receptor in a human or animal patient in need thereof, comprising administering to the human or animal patient an effective quantity of a compound of formula (I).

Another aspect of the present invention provides the use of a compound of formula (I) in preparing a medicament for human or animal administration, useful for antagonising the activity of the ORL-1 receptor.

The compounds of the invention are therefore useful in the therapy and/or prophylaxis of all those illnesses dependent on activation of the ORL-1 receptor. Accordingly they can be used as analgesics in man or animals in treating or preventing, for example, acute pain, chronic neuropathic or inflammatory pain, including post-herpes neuralgia, neuralgia, diabetic neuropathy and post-infarct pain; visceral pain including that associated with irritable bowel syndrome, dysmenorrhea, and hyperreflexia of the bladder; osteoarthritis, back pain, labour pain in childbirth.

Said compounds can further be useful in the treatment or prophylaxis of gastrointestinal disorders including irritable bowel syndrome, and symptoms associated with non-ulcerous dyspepsia and gastro-oesophageal reflux; diseases of the immune system; dysfunctions of the cardiovascular system such as infarct, congestive cardiac insufficiency and pathologies associated with alterations of arterial pressure; diseases of the excretory system, such as altered diuresis, water homeostasis and sodium excretion, syndrome of inappropriate anti-diuretic hormone secretion (SIADH); sexual dysfunctions including impotence and frigidity; cirrhosis with ascites.

These compounds can also be useful in the treatment or prophylaxis of disorders of the respiratory tract such as cough, asthma, adult respiratory distress syndrome (ARDS), altered pulmonary function, including chronic obstructive pulmonary disease.

Compounds of the invention are further useful in the treatment of central nervous system disorders where ORL-1 receptors are involved. In particular in the treatment or prevention of major depressive disorders including bipolar depression, unipolar depression, single or recurrent major depressive episodes with or without psychotic or catatonic features, catatonic features, melancholic features, atypical features or postpartum onset, the treatment of anxiety and the treatment of panic disorders. The term anxiety includes anxiety disorders, such as panic disorders with or without agoraphobia, agoraphobia, phobias, for example, social phobias or agoraphobia, obsessive-compulsive disorder, stress disorders including post-traumatic stress disorders, generalised anxiety disorders, acute stress disorders and mixed anxiety-depression disorders. Other mood disorders encompassed within the term major depressive disorders include dysthymic disorder with early or late onset and with or without atypical features, neurotic depression, post traumatic stress disorders, post operative stress and social phobia; dementia of the Alzheimer's type, with early or late onset, with depressed mood; vascular dementia with depressed mood; mood disorders induced by alcohol, amphetamines, cocaine, hallucinogens, inhalants, opioids, phencyclidine, sedatives, hypnotics, anxiolytics and other substances; schizoaffective disorder of the depressed type; and adjustment disorder with depressed mood. Major depressive disorders may also result from a general medical condition including, but not limited to, myocardial infarction, diabetes, miscarriage or abortion, etc.

Compounds of the invention are also useful in the treatment or prevention of dementia as such, e.g. vascular dementia and dementia associated with AIDS; they are further effective in treating or preventing motor damage and neurodegeneration due to Alzheimer's disease; senile dementia, Parkinson's disease, disorders due to defective motor coordination or other neurodegenerative pathologies.

Compounds of the invention are also useful in the treatment or prevention of epilepsy; schizophrenic disorders including paranoid schizophrenia, disorganised schizophrenia, catatonic schizophrenia, undifferentiated schizophrenia, residual schizoprenia.

Compounds of the invention are also useful for the treatment of dysfunction of appetite and food intake and in circumstances such as anorexia, anorexia nervosa bulimia, and metabolic disorders such as obesity.

Compounds of the invention are also useful in the treatment of sleep disorders including dysomnia, insomnia, sleep apnea, narcolepsy, and circadian rhythmic disorders.

Compounds of the invention are also useful in the treatment or prevention of cognitive disorders. Cognitive disorders include dementia, amnestic disorders , memory loss, and cognitive disorders not otherwise specified. Furthermore compounds of the invention are also useful as memory and/or cognition enhancers in healthy humans with no cognitive and/or memory deficit.

Compounds of the invention are also useful in the treatment of drug abuse, tolerance to and dependence on a number of substances. For example, they are useful in the treatment of dependence on nicotine, alcohol, caffeine, phencyclidine (phencyclidine like compounds), or in the treatment of tolerance to and dependence on opiates (e.g. cannabis, heroin, morphine) or benzodiazepines; in the treatment of cocaine, sedative ipnotic, amphetamine or amphetamine- related drugs (e.g. dextroamphetamine, methylamphetamine) addiction or a combination thereof.

The compounds of the present invention show an elevated ORL-1 binding affinity; in addition they show a remarkably low inhibition of cytochrome P450 activity, in particular with respect to the 1A2, 2C9, 2C19, 2D6 and 3A4 isoforms, which are the main responsible for the metabolism of the majority of drugs currently on the market. Each modification of the activity of these enzymes may lead to the socalled "drug-drug interaction", a phenomenon that occurs when the kinetic disposition of a drug is modified by another drug. The remarkable low inhibitory power of the present compounds towards the cytochrome P450 isoenzymes makes them particularly adapted to perform as ORL-1 ligands with low or negligible propensity to give drug-drug interaction in combined therapies and hence reducing the risk of unwanted side-effects associated to the altered metabolism of the co-administered drug.

The compounds of formula (I) can be prepared in the form of salts or hydrates. Suitable salts are pharmaceutically acceptable salts. Suitable hydrates are pharmaceutically acceptable hydrates.

An effective quantity of compound of the invention depends on factors such as the nature or seriousness of the illness or illnesses to be treated and on the weight of the patient. In all cases a unit dose normally contains from 0.1 to 50 mg, for example from 0.5 to 10 mg, of the compound. Unit doses are normally administered one or more times per day, for example, 2, 3 or 4 times a day, in particular from 1 to 3 times per day, so that the total daily dose is normally, for an adult of 70 kg, between 0.1 and 50 mg, for example between 0.1 and 5 mg, i.e. in the approximate range of 0.001 to 1 mg/kg/day, in particular between 0.005 and 0.2 mg/kg/day. For oral or parenteral administration, it is highly preferred that the compound be administered in the form of unit dose composition for example, in the form of unit dose oral or parenteral composition.

These compositions are prepared by mixing and are suitably adapted to oral or parenteral administration, and as such can be in the form of tablets, capsules, oral preparations, powders, granules, lozenges, reconstitutable powders, injectable or infusible liquid solutions, suspensions or suppositories.

Tablets and capsules for oral administration are normally presented in unit dose form, and contain conventional excipients such as binders, fillers, diluents, compressing agents, lubricants, detergents, disintegrants, colorants, aromas and wetting agents. The tablets can be covered by methods well known in the art.

Suitable fillers include cellulose, mannitol, lactose and other similar agents. Suitable disintegrants include starch, polyvinylpyrrolidone and starch derivatives such as sodium glycolate starch. Suitable lubricants include, for example, magnesium stearate. Suitable wetting agents include sodium laurylsulfate.

These solid oral compositions can be prepared by conventional methods of mixing, filling or compression. The mixing operations can be repeated to disperse the active component in compositions containing large quantities of fillers. These operations are conventional.

Oral liquid preparations can be in the form, for example, of aqueous or oily suspensions, solutions, emulsions, syrups, or elixirs, or can be presented as a dry product for reconstitution with water or with a suitable carrier before use. These liquid preparations can contain conventional additives such as suspending agents, for example sorbitol, syrup, methylcellulose, gelatin, hydroxyethylcellulose, carboxymethylcellulose, aluminium stearate gel or hydrogenated edible fats, emulsifying agents, for example lecithin, sorbitan monooleate, or acacia; nonaqueous carriers (which can include edible oils), for example almond oil, fractionated coconut oil, oily esters such as glycerine esters, propylene glycol, or ethyl alcohol; preservatives, for example methyl or propyl p-hydroxybenzoate or sorbic acid, and if desired, conventional aromas or colorants.

Oral formulations also include conventional slow-release formulations, such as tablets or granules having an enteric coating.

For parenteral administration, fluid dose units can be prepared, containing the compound and a sterile carrier. The compound, depending on the carrier and the concentration, can be suspended or dissolved. Parenteral solutions are normally prepared by dissolving the compound in a carrier and sterilizing by means of a filter, before filling suitable vials or ampoules and sealing. Advantageously, adjuvants such as local anaesthetics, preservatives and buffer agents can also be dissolved in the carrier. To increase stability, the composition can be frozen after filling the vial and removing the water under vacuum. Parenteral suspensions are prepared substantially in the same manner, with the difference that the compound can be suspended in the carrier instead of dissolved, and be sterilized by exposure to ethylene oxide before suspension in the sterile carrier. Advantageously, a surfactant or a wetting agent can be included in the composition to facilitate uniform distribution of the compound of the invention.

As in common practise, the compositions are normally accompanied by written or printed instructions, for use in the treatment in question.

Consequently, another aspect of the present invention also provides a pharmaceutical composition comprising a compound of formula (I), or a pharmaceutically suitable salt or hydrate thereof, and a pharmaceutically acceptable carrier.

The invention will now be illustrated by means of the following non-limiting examples.

### EXPERIMENTAL PART

### General Preparation 1

3.6 mmol of the appropriate phenylpiperidine were dissolved in 13 mL of MeOH/dioxane 8:2 mixture respectively, then 0.297 mL (3.96 mmol) of 37% aqueous formaldehyde and 0.246 mL (4.32 mmol) of glacial acetic acid were added. After stirring at room temperature for 20 min, 2-phenylindole (904 mg, 4.68 mmol) dissolved in 36 mL of 8:2 MeOH/dioxane mixture was added and the reaction mixture was stirred overnight at rt. The solvent was evaporated, the residue was taken up in AcOEt/H₂O, basified with conc. NH₄OH, extracted, dried and the crude compound was purified by chromatography.

The following compounds were prepared according to General Preparation 1
3-[4-(2-Chloro-6-fluoro-phenyl)-piperidin-1-ylmethyl]-2-phenyl-1H-indole
3-[4-(2,6-Difluoro-phenyl)-piperidin-1-ylmethyl]-2-phenyl-1H-indole

### General Preparation 2

3.92 mmol of the appropriate phenylpiperidine were dissolved in 10 mL of EtOH/dioxane mixture 8:2 respectively, then 0.44 mL (5.88 mmol) of 37% aqueous formaldehyde and 4 mL of AcOH were added. The reaction mixture was stirred 20 min at rt, then a solution of ethyl indole-2-carboxylate (1.11 g, 5.88 mmol) in 25 mL of 8:2 EtOH/dioxane mixture was added dropwise. The reaction mixture was heated to 80°C for 12 h, then the solvents were evaporated, the residue was taken up with water and AcOEt, basified with conc. NH₄OH, extracted, dried and evaporated. The resulting crude compound was purified by chromatography.

The following compounds were prepared according to General Preparation 2
3-[4-(2-Chloro-6-fluoro-phenyl)-piperidin-1-ylmethyl]-1H-indole-2-carboxylic acid ethyl ester
3-[4-(2,6-Difluoro-phenyl)-piperidin-1-ylmethyl]-1H-indole-2-carboxylic acid ethyl ester

### General Procedure A

Under a nitrogen atmosphere, 0.37 mmol of amine were dissolved in 0.5 mL of dry toluene, then 0.185 mL (0.37 mmol) of a 2M solution of AlMe₃ in toluene were added dropwise at room temperature. After stirring for 45 min, a solution of 0.185 mmol of the appropriate compound obtained from General Preparation 2, in 1 mL of dry toluene was added dropwise. The reaction mixture was heated to reflux for 90 min, then, after cooling to room temperature, water and AcOEt were added. The organic phase was collected, washed with brine, dried and the solvent was removed *in vacuo.*

The crude products were purified by chromatography, yielding the compounds described in Examples 1-6.

### Example 1

### 2-(4-{3-[4-(2-Chloro-6-fluoro-phenyl)-piperidin-1-ylmethyl]-1H-indole-2-carbonyl}-piperazin-1-yl)-1-morpholin-4-yl-ethanone

NMR (300 MHz, DMSOd₆, δ ppm): 11.34 (s 1 H); 7.73 (d, 1 H); 7.35 (d, 1H); 7.32-7.22 (m, 2H); 7.20-7.09 (m, 2H); 7.04 (m, 1 H); 3.68 (s, 2H); 3.65-3.36 (m, 17H); 3.08 (m, 1 H); 2.95 (m, 2H); 2.13-1.93 (m, 5H); 1.57 (m, 2H).
ESI Pos, 3.2KV, 20V, 400°C MS (m/z): 582.2 (MH⁺).

### Example 2

### 2-(4-{3-[4-(2,6-Difluoro-phenyl)-piperidin-1-ylmethyl]-1H-indole-2-carbonyl}-piperazin-1-yl)-1-morpholin-4-yl-ethanone

NMR (300 MHz, DMSOd₆ +TFA, δ ppm): 12.12 (s 1 H); 9.48 (s br, 1 H); 7.92 (d, 1 H); 7.53 (d, 1 H); 7.38-7.19 (m, 3H); 7.06 (m, 2H); 4.56 (d, 2H); 4.37 (s, 2H); 3.67-3.09 (m, 19H); 2.35-2.17 (m, 2H); 1.92 (m, 2H).
ESI Pos, 3.2KV, 20V, 400°C MS (m/z): 566.3 (MH⁺).

### Example 3

### N-(1-{3-[4-(2-Chloro-6-fluoro-phenyl)-piperidin-1-ylmethyl]-1 H-indole-2-carbonyl}-pyrrolidin-3-yl)-N-methyl-acetamide

NMR (300 MHz, DMSOd₆ 373K, δ ppm): 10.93 (s, 1 H); 7.76 (d, 1 H); 7.38 (d, 1 H); 7.28-6.99 (m, 5H); 4.84 (m, 1 H); 4.19 (d, 1 H); 3.78 (s, 2H); 3.69 (m, 2H); 3.59-3.39 (m, 2H); 3.24-2.80 (m, 7H); 2.19-2.04 (m, 6H); 2.02 (s, 3H).
ESI Pos, 3.2KV, 20V, 400°C MS (m/z): 511.2 (MH⁺).

### Example 4

### N-(1-{3-[4-(2,6-Difluoro-phenyl)-piperidin-1-ylmethyl]-1H-indole-2-carbonyl}-pyrrolidin-3-yl)-N-methyl-acetamide

NMR (300 MHz, DMSOd₆ 373K, δ ppm): 10.94 (s, 1 H); 7.77 (d, 1 H); 7.38 (d, 1 H); 7.27-7.01 (m, 5H); 4.85 (m, 1 H); 4.18 (d, 1 H); 3.78 (s, 2H); 3.68 (m, 2H); 3.59-3.39 (m, 2H); 3.23-2.82 (m, 7H); 2.19-2.04 (m, 6H); 2.03 (s, 3H).
ESI Pos, 3.2KV, 20V, 400°C MS (m/z): 495.1 (MH⁺).

### Example 5

### {3-[4-(2-Chloro-6-fluoro-phenyl)-piperidin-1-ylmethyl]-1H-indol-2-yl}-{4-[2-(2-hydroxy-ethoxy)-ethyl]-piperazin-1-yl}-methanone

NMR (300 MHz, CDCl₃+D₂O+Na₂CO₃, δ ppm): 7.82 (d, 1 H); 7.37 (d, 1 H); 7.32 (dd, 1H); 7.17-7.00 (m, 3H); 6.89 (m, 1H); 3.78 (s, 2H); 3.77-3.53 (m, 10H); 3.13 (m, 1 H); 3.01 (m, 2H); 2.63 (dd, 2H); 2.57 (m, 4H); 2.23-1.98 (m, 4H); 1.61 (m, 2H).
ESI Pos, 3.2KV, 20V, 400°C MS (m/z): 543.1 (MH⁺).

### Example 6

### {3-[4-(2,6-Difluoro-phenyl)-piperidin-1-ylmethyl]-1H-indol-2-yl}-{4-[2-(2-hydroxy-ethoxy)-ethyl]-piperazin-1-yl}-methanone

NMR (300 MHz, CDCl₃, δ ppm): 9.31 (s, 1 H); 7.82 (d, 1 H); 7.36 (d, 1 H); 7.21 (m, 1H); 7.16-7.01(m, 2H); 6.78 (dd, 2H); 3.76 (s, 2H); 3.74-3.56 (m, 10H); 3.00 (m, 2H); 2.92 (m, 1 H); 2.62 (dd, 2H); 2.56 (m, 4H); 2.16-1.99 (m, 5H); 1.61 (m, 2H);
ESI Pos, 3.2KV, 20V, 400°C MS (m/z): 527.3 (MH⁺).

### Example 7

### 2-[3-[4-(2-Chloro-6-fluoro-phenyl)-piperidin-1-ylmethyl]-2-(morpholine-4-carbonyl)-indol-1-yl]-acetamide

### Step 1)

Under a nitrogen atmosphere 0.09 mL (1.04 mmmol) of morpholine were dissolved in 1.4 mL of dry toluene, then 0.52 mL (1.04 mmol) of a 2M solution of AlMe₃ in toluene were added dropwise at room temperature. After stirring for 45 min a solution of 215.9 mg (0.52 mmol) of 3-[4-(2-chloro-6-fluoro-phenyl)-piperidin-1-ylmethyl]-1H-indole-2-carboxylic acid ethyl ester (obtained as described in General Preparation 2) in 3.5 mL of dry toluene was added dropwise. The reaction mixture was heated at 80°C for 90 min and then the reaction was cooled to room temperature. Water and AcOEt were added, the organic phase was separated and washed with brine, dried and the solvent was removed in vacuo, yielding 269 mg of {3-[4-(2-chloro-6-fluoro-phenyl)-piperidin-1-ylmethyl]-1H-indol-2-yl}-morpholin-4-yl-methanone, used in step 2 without further purification.

### Step 2)

Under a nitrogen atmosphere, 269 mg of {3-[4-(2-chloro-6-fluoro-phenyl)-piperidin-1-ylmethyl]-1H-indol-2-yl}-morpholin-4-yl-methanone (crude compound obtained from step 1) were dissolved in 5 mL of dry DMF; the resulting solution was cooled to 0°C and 47 mg (1.17 mmol) of NaH (60% dispersion in mineral oil) were added; the reaction mixture was stirred at 0°C for 30 min, then 0.17 mL (1.14 mmol) of tert-butyl bromoacetate were added dropwise; the reaction mixture was allowed to warm to room temperature and stirred overnight. After cooling to 0°C the reaction was carefully quenched with water, then the reaction mixture was extracted with AcOEt and washed with brine; the organic phase was dried with Na₂SO₄ and the solvent was removed in vacuo, yielding 380 mg of [3-[4-(2-chloro-6-fluoro-phenyl)-piperidin-1-ylmethyl]-2-(morpholine-4-carbonyl)-indol-1-yl]-acetic acid tert-butyl ester used in step 3 without further purification.

### Step 3)

2 mL of TFA were added to a solution of 380 mg of [3-[4-(2-chloro-6-fluorophenyl)-piperidin-1-ylmethyl]-2-(morpholine-4-carbonyl)-indol-1-yl]-acetic acid tert-butyl ester (crude compound from step 2) in 5 mL of CH₂Cl₂. The mixture was stirred at room temperature overnight, then the solvent was removed in vacuo, yielding 295 mg of [3-[4-(2-chloro-6-fluoro-phenyl)-piperidin-1-ylmethyl]-2-(morpholine-4-carbonyl)-indol-1-yl]-acetic acid trifluoroacetate used in step 4 without further purification.

### Step 4)

295 mg of [3-[4-(2-chloro-6-fluoro-phenyl)-piperidin-1-ylmethyl]-2-(morpholine-4-carbonyl)-indol-1-yl]-acetic acid trifluoroacetate (crude compound from step 3) were dissolved, under nitrogen atmosphere, in 4 ml of dry DMF and 186 mg (1.15 mmol) of N,N'-carbonyldiimidazole were added. The reaction mixture was stirred at room temperature for 3h, then 2 mL of conc. NH₄OH were added and the reaction mixture was stirred overnight at rt. AcOEt was added to the reaction mixture, which was then basified with 1 M NaOH; the organic phase was collected, washed with brine, dried with Na₂SO₄ and the solvent was removed in vacuo. The crude compound was purified by cromatography, eluting with CH₂Cl₂ and then with a mixture CH₂Cl₂/MeOH/conc. NH₄OH (98:2:0.1 respectively), yielding 93 mg of the title compound.
NMR (300 MHz, DMSOd₆ 373K, δ ppm): 7.77 (d, 1 H); 7.38 (d, 1 H); 7.28-7.18 (m, 3H); 7.15-7.05 (m, 2H); 6.97 (s br, 2H); 4.75 (s, 2H); 3.68 (s, 2H); 3.68-3.40 (m, 8H); 3.23-2.96 (m, 3H); 2.22-2.02 (m, 4H); 1.61 (m, 2H).
ESI Pos, 3.2KV, 20V, 400°C MS (m/z): 513.2 (MH⁺).

Compound of Example 8 was obtained following procedure described in Example 7, starting from 3-[4-(2,6-difluoro-phenyl)-piperidin-1-ylmethyl]-1H-indole-2-carboxylic acid ethyl ester, obtained as described in General Preparation 2.

### Example 8

### 2-[3-[4-(2,6-Difluoro-phenyl)-piperidin-1-ylmethyl]-2-(morpholine-4-carbonyl)-indol-1-yl]-acetamide

NMR (300 MHz, DMSOd₆ 353K, δ ppm): 7.75 (d, 1 H); 7.38 (d, 1 H); 7.30-7.17 (m, 2H); 7.10 (dd, 1 H); 7.06 (s br, 2H); 6.98 (dd, 2H); 4.76 (s, 2H); 3.76-3.38 (m, 11 H); 2.96 (m, 2H); 2.06 (m, 4H); 1.62 (m, 2H).
ESI Pos, 3.2KV, 20V, 400°C MS (m/z): 497.2 (MH⁺).

### Example 9

### {3-[4-(2-Chloro-6-fluoro-phenyl)-piperidin-1-ylmethyl]-1H-Indol-2-yl}-(1S,4S)-2-oxa-5-aza-bicyclo[2.2.1]hept-5-yl-methanone

To a suspension of 204 mg (0.492 mmol) of 3-[4-(2-chloro-6-fluoro-phenyl)-piperidin-1-ylmethyl]-1H-indole-2-carboxylic acid ethyl ester (obtained as described in General Preparation 2) in EtOH (2.5 mL), 1 mL of 1 N sodium hydroxide solution was added and the mixture was stirred at 80°C for 2 h. The solvent was evaporated under reduced pressure and the pale yellow solid obtained (199 mg) was suspended in SOCl₂ (2 mL) and the reaction mixture was stirred at 50°C for 2 h. Excess SOCl₂ was evaporated under reduced pressure and the light brown solid obtained was suspended in dry DMF (1 mL) and added dropwise at 0°C to a solution of (1S,4S)-2-Oxa-5-aza-bicyclo[2.2.1]heptane hydrochloride (133 mg, 0.98 mmol) and triethylamine (0.34 mL, 2.45 mmol) in CH₂Cl₂ (3 mL). The mixture was stirred at room temperature overnight, then it was washed with water (2x5 mL) and with 1 N NaOH solution (2x5 mL). Organic layers were collected, dried over Na₂SO₄ and evaporated under reduced pressure. The crude product obtained was purified by flash chromatography (CH₂Cl₂/MeOH/NH₄OH 96:3.5:0.5 to 93:6:1 respectively) yielding 25 mg of the title compound.
NMR (300 MHz, CDCl₃, δ ppm): 11.52 (s br, 1 H); 7.72 (d, 1 H); 7.60 (d br, 1 H); 7.24 (dd, 1 H); 7.13-7.05 (m, 3H); 6.91 (m, 1 H); 4.86 (m, 1 H); 4.62 (m, 1 H); 4.36-4.05 (m, 2H); 4.00 (d, 1 H); 3.79 (m, 1 H); 3.57 (m, 1H); 3.26 (m, 3H); 2.88-2.22 (m, 3H); 2.05 (m, 1 H); 1.90 (m, 1 H); 1.77 (m, 4H).
ESI Pos, 3.2KV, 20V, 400°C MS (m/z): 468.04 (MH⁺).

Compound of Example 10 was prepared according to procedure described in Example 9, starting from 3-[4-(2,6-difluoro-phenyl)-piperidin-1-ylmethyl]-1H-indole-2-carboxylic acid ethyl ester, obtained as described in General Preparation 2.

### Example 10

### {3-[4-(2,6-Difluoro-phenyl)-piperidin-1-ylmethyl]-1 H-indol-2-yl}-(1S,4S)-2-oxa-5-aza-bicyclo[2.2.1]hept-5-yl-methanone hydrochloride

NMR (300 MHz, CDCl₃, δ ppm): 11.51 (s br, 1 H); 11.50 (s br, 1 H); 7.77 (m, 2H); 7.18 (m, 3H); 6.84 (m, 2H); 4.91 (m, 1 H); 4.69 (m, 1 H); 4.54 (m, 2H); 4.00 (m, 1 H); 3.85 (m, 1 H); 3.64 (m, 1 H); 3.45 (m, 3H); 3.20 (m, 3H); 2.24 (m, 2H); 1.94 (m, 4H). ESI Pos, 3.2KV, 20V, 400°C MS (m/z): 452.1 (MH⁺).

### Example 11

### 2-{3-[4-(2-Chloro-6-fluoro-phenyl)-piperidin-1-ylmethyl]-2-methyl-indol-1-yl}-N-(1-methyl-piperidin-4-yl)-acetamide

### Step 1)

A solution of 2-methyl-1H-indole (2 g ,15.3 mmol) in 15ml of dry DMF was cooled to 0°C and 403 mg (16.8 mmol) of NaH (60% dispersion in mineral oil) were added; the reaction mixture was stirred at 0°C for 30 min, then 4.5 mL (30.6 mmol) of tert-butyl bromoacetate were added; the reaction mixture was allowed to warm to room temperature and stirred overnight. After cooling to 0°C the reaction was carefully quenched with water, then the reaction mixture was extracted with AcOEt and washed with brine; the organic phase was dried with Na₂SO₄ and the solvent was removed in vacuo, yielding 3.1 g of *tert*-butyl 2-(2-methyl-1 H-indol-1-yl)acetate used in step 2 without further purification.

### Step 2)

A solution of 151 mg (0.71 mmol) of 4-(2-chloro-6-fluorophenyl)piperidine in 2.5 mL of MeOH/dioxane 8:2 mixture, was treated with 0.063 mL (0.78 mmol) of 37% aqueous formaldehyde and 0.02 mL (0.852 mmol) of glacial acetic acid. After stirring at room temperature for 20 min, 226 mg (0.92 mmol) of *tert*-butyl 2-(2-methyl-1 H-indol-1-yl)acetate (compound obtained in step 1) dissolved in 6.9 mL of MeOH/dioxane 8:2 mixture were added and the reaction mixture was stirred overnight at room temperature. The solvent was evaporated and the residue was taken up in AcOEt/H₂O, basified with concd. NH₄OH and extracted. The organic phase was dried with Na₂SO₄ and the solvent was removed in vacuo. The crude compound was purified by cromatography eluting with CH₂Cl₂ and then with a mixture CH₂Cl₂/MeOH/concd. NH₄OH (95:5:0.5 respectively), yielding 260 mg of *tert*-butyl 2-(3-((4-(2-chloro-6-fluorophenyl)piperidin-1-yl)methyl)-2-methyl-1H-indol-1-yl)acetate.

### Step 3)

A solution of 260 mg (0.60 mmol) of *tert*-butyl 2-(3-((4-(2-chloro-6-fluorophenyl)piperidin-1-yl)methyl)-2-methyl-1H-indol-1-yl)acetate (compound from step 2) in 4 mL of CH₂Cl₂ was treated with 1 mL of TFA and the mixture was stirred at room temperature overnight. The solvent was removed in vacuo, yielding 174 mg of 2-(3-((4-(2-chloro-6-fluorophenyl)piperidin-1-yl)methyl)-2-methyl-1H-indol-1-yl)acetic acid trifluoroacetate which was used in step 4 without further purification.

### Step 4)

The crude 2-(3-((4-(2-chloro-6-fluorophenyl)piperidin-1-yl)methyl)-2-methyl-1H-indol-1-yl)acetic acid trifluoroacetate from step 3 (174 mg) was dissolved in 4 mL of dry DMF under a nitrogen atmosphere, then 215 mg (1.3 mmol) of N,N'-carbonyldiimidazole were added. The reaction mixture was stirred at room temperature for 3h, then 140 mg (1.23 mmol) of 1-methylpiperidin-4-amine were added and the reaction mixture was stirred overnight at room temperature. AcOEt was added to the reaction mixture, which was then basified with 1M NaOH; the organic phase was collected, washed with brine, dried with Na₂SO₄ and the solvent was removed in vacuo.

The crude compound was purified by cromatography eluting with CH₂Cl₂ and then with a mixture CH₂Cl₂/MeOH/conc. NH₄OH (95:5:0.5 respectively), yielding 140 mg of the title product.
NMR (300 MHz, DMSOd₆ 353K, δ ppm): 7.63-7.56 (m, 2H); 7.31-7.21 (m, 3H); 7.15-6.95 (m, 3H); 4.72 (s, 2H); 3.64 (s, 2H); 3.54 (m, 1 H); 3.19-2.96 (m, 3H); 2.62 (m, 2H); 2.37 (s, 3H); 2.15 (s, 3H); 2.13-1.91 (m, 6H); 1.78-1.38 (m, 6H).
ESI Pos, 3.2KV, 20V, 400°C MS (m/z): 511.2 (MH⁺).

Compounds described in Examples 12-15 were obtained following steps 1, 3 and 4 described in Example 11, starting from 3-[4-(2-chloro-6-fluoro-phenyl)-piperidin-1-ylmethyl]-2-phenyl-1 H-indole or 3-[4-(2,6-difluoro-phenyl)-piperidin-1-ylmethyl]-2-phenyl-1 H-indole (obtained as described in General Preparation 1).

### Example 12

### 2-{3-[4-(2-Chloro-6-fluoro-phenyl)-piperidin-1-ylmethyl]-2-phenyl-indol-1-y}-1-morpholin-4-yl-ethanone

NMR (300 MHz, DMSOd₆ 353K, δ ppm): 7.79 (d, 1 H); 7.56-7.42 (m, 5H); 7.31 (d, 1 H); 7.27-7.21 (m, 2H); 7.20-7.05 (m, 3H); 4.87 (s, 2H); 3.57 (s, 2H); 3.51 (m, 4H); 3.41 (m, 4H); 3.11-2.93 (m, 3H); 2.13-1.89 (m, 4H); 1.57 (m, 2H).
ESI Pos, 3.2KV, 20V, 400°C MS (m/z): 546.2 (MH⁺).

### Example 13

### 2-{3-[4-(2,6-Difluoro-phenyl)-piperidin-1-ylmethyl]-2-phenyl-indol-1-yl}-l-morpholin-4-yl-ethanone

NMR (300 MHz, DMSOd₆ 373K, δ ppm): 7.80 (d, 1 H); 7.55-7.41 (m. 5H); 7.31 (d, 1 H); 7.25 (dd, 1 H); 7.16 (ddd, 1 H); 7.09 (ddd, 1 H); 6.96 (dd, 2H); 4.86 (s, 2H); 3.58 (s, 2H); 3.51 (m, 4H); 3.40 (m, 4H); 3.00-2.88 (m, 3H); 1.98 (m, 4H); 1.60 (m, 2H).
ESI Pos, 3.2KV, 20V, 400°C MS (m/z): 530.12 (MH⁺).

### Example 14

### 2-{3-[4-(2-Chloro-6-fluoro-phenyl)-piperidin-1-ylmethyl]-2-phenyl-indol-1-yl}-1-(4-methyl-piperazin-1-yl)-ethanone

NMR (300 MHz, DMSOd₆ 373K, δ ppm): 7.79 (d, 1H); 7.55-7.41 (m, 5H); 7.28 (d, 1 H); 7.26-7.20 (m, 2H); 7.19-7.02 (m, 3H); 4.84 (s, 2H); 3.58 (s, 2H); 3.39 (m, 4H); 3.10 (m, 1 H); 2.98 (m, 2H); 2.22 (m, 4H); 2.19 (s, 3H); 2.15-1.91 (m, 4H); 1.57 (m, 2H).
ESI Pos, 3.2KV, 20V, 400°C MS (m/z): 559.2 (MH⁺).

### Example 15

### 2-{3-[4-(2,6-Difluoro-phenyl)-piperidin-1-ylmethyl]-2-phenyl-indol-1-yl}-1-(4-methyl-piperazin-1-yl)-ethanone

NMR (300 MHz, DMSOd₆ 373K, δ ppm): 7.79 (d, 1 H); 7.58-7.42 (m, 5H); 7.29 (d, 1 H); 7.23 (m, 1 H); 7.15 (ddd, 1 H); 7.09 (ddd, 1 H); 6.95 (dd, 2H); 4.84 (s, 2H); 3.57 (s, 2H); 3.39 (m, 4H); 3.00-2.85 (m, 3H); 2.22 (m, 4H); 2.19 (s, 3H);1.98 (m, 4H); 1.60 (m, 2H).
ESI Pos, 3.2KV, 20V, 400°C MS (m/z): 543.11 (MH⁺).

The compound described in Example 16 was obtained following procedure described in Example 11.

### Example 16

### 2-{3-[4-(2,6-Difluoro-phenyl)-piperidin-1-ylmethyl]-2-methyl-indol-1-yl}-1-(4-methyl-piperazin-1-yl)-ethanone

NMR (300 MHz, DMSOd₆ 373K, δ ppm): 7.58 (m, 1 H); 7.31-7.20 (m, 2H); 7.07-6.93 (m, 4H); 5.02 (s, 2H); 3.66 (s, 2H); 3.56 (dd, 4H); 3.03 (m, 2H); 2.93 (m, 1 H); 2.37 (dd, 4H); 2.32 (s, 3H); 2.25 (s, 3H); 2.15-1.94 (m, 4H); 1.65 (m, 2H).
ESI Pos, 3.2KV, 20V, 400°C MS (m/z): 481.19 (MH⁺).

### Pharmacological tests

### Receptor binding studies

The receptor binding studies were carried out on 96-well plates; the incubation medium was Tris HCl pH 7.4 (4°C) containing MgCl₂ (5 mM), EGTA (0.2 mM), BSA (0.1 %), with a final volume of 1.0 ml, using as radioligand [³H]-AcRYYRWK-NH₂ (Amersham, 103 Ci/mmol). The samples were incubated at 37°C for 120 min. and were then filtered off via Whatman GF/B filters pre-treated with 0.2% polyethylenimine. The filters were washed three times with Tris HCl buffer pH 7.4 (4°C). The radioactivity present on the filters was measured using a Packard Top Count microplate scintillation counter.

### Preparation of membrane for the GTPγS binding test

The entire process was carried out at 4°C. The buffer used consisted of Tris HCl 10 mM, EDTA 0.1 mM, pH 7.4 (4°C) (T.E.).

The cells removed from the culture flask are centrifuged at low speed to remove the growth medium.
1. Resuspend the pellets (a 175 cm² T flask in 0.5-1 ml T.E.).
2. Homogenize the cells using an Ultra-Turrax
3. Centrifuge the homogenate at 1,500 rpm for 10 min at 4°C.
4. Discard the pellets P1
5. Centrifuge the supernatant at 14,000 rpm for 30 min.
6. Discard the supernatant.
7. Resuspend the pellets P2 by suction (microsomal fraction) in 200 ml of T.E. and preserve frozen at -80°C.

To estimate the proteins, dilute the preparation 3 x in T.E. and assay against standard BSA curve 0-2 mg/ml in T.E.. The protein concentration is normally between 1 and 4 mg/ml. The typical yield is 1 mg of proteins per 175 cm² T flask at confluence.

### Binding tests [³⁵S]-GTPγS

The tests were carried out in a 96-well plate using the method modified by Wieland and Jacobs (Methods Enzymol., 1994, 237, 3-13). The membranes (10 µg per well) and the SPA granules of wheat germ agglutinin (Amersham Pharmacia) (0.5 mg per well) were pre-mixed in buffer solution (HEPES 20 mM, NaCl 100 mM, MgCl₂ 10 mM, pH 7.4, 4°C) and pre-incubated with 10 µM GDP. Increasing concentrations of the compounds to be tested were then incubated with the membrane/granule mixture for 30 min at ambient temperature. 0.3 nM [³⁵S]-GTP_{γ}S (1170 Ci/mmol, Amersham) and the ORL-1 agonist were then added. The total volume of the assay is 100 µl per well. The plates are then incubated at ambient temperature for 30 min under agitation and then centrifuged at 1500 g for 5 min. The quantity of [³⁵S]-GTPγS bound to the membranes was determined by a Wallac microbeta 1450-Trilux scintillation counter.

The activity of the compound is evaluated as inhibition of [³⁵S]-GTPγS binding stimulation induced by the agonist.

The pIC50 values are determined as the concentration of compound which causes a 50% inhibition of the agonist response.

### Electrically stimulated guinea pig ileum (GPI) assay

Compounds of the invention are evaluated for ORL-1 functional activity in electrically stimulated guinea pig ileum, in agreement with the experimental protocol described in Calò et. al., Br. J. Pharmacol. 129, 1183, (2000).

The ORL-1 antagonist activity (pK_{b}) is evaluated against the endogenous ORL-1 agonist N/OFQ.

### Interaction with Cytochrome P450 isoenzymes

The assay was performed in a 96-well microtiter plates as described in Stresser et al., Drug Metab. Dispos. 28, 1440 (2000). Microsomes from baculovirus-insect cells (Supersomes, Gentest Corporation, Woburn, MA) expressing human cytochrome P450 isoforms (CYP1A2, CYP2C9, CYP2C19, CYP2D6 and CYP3A4) were utilized. Test compounds or standard inhibitors (dissolved in DMSO or acetonitrile) were serially diluted in a solution containing 16.3 uM NADP⁺, 0.83 mM glucose-6-phosphate, 0.83 mM MgCl₂, 0.4 U/mL of glucose-6-phosphate dehydrogenase and 0.1 mg/mL microsomal protein prepared from wild-type baculovirus-infected insect cells. Appropriate control wells without inhibitor and without microsomes were also added. The plates were then pre-warmed at 37°C for 10 min, and the reaction was started by adding pre-warmed enzyme/substrate mix (cytochrome P450 isoforms with their specific substrates in a 0.35 M potassium phosphate buffer at pH 7.4). Specific substrates were 3-cyano-7-ethoxycoumarin (CEC) for CYP1A2 and CYP2C19, 7-methoxy-4-trifluoromethylcoumarin (MFC) or dibenzylfluorescein (DBF) for CYP2C9, 3-[2-(N,N-diethyl-N-methylamino)ethyl]-7-methoxy-4-methylcoumarin (AMMC) for CYP2D6 and 7-benzyloxy-4-(trifluoromethyl)-coumarin (BFC) for CYP3A4. Reaction was terminated at various times, depending on the assays, by addition of a 4:1 acetonitrile:0.5 M Tris base solution. Known inhibitors for each isoenzyme (furafylline for CYP1A2, sulfaphenazole for CYP2C9, tranylcypromine for CYP2C19, quinidine for CYP2D6 and ketoconazole for CYP3A4) were tested in all assays as positive controls. Plates were read on a fluorometer (Fluoroskan Ascent, ThermoElectron Corporation, Helsinki, Finland) at the appropriate emission/excitation wavelengths. Median inhibitory concentration (IC₅₀) was determined by nonlinear regression (GraFit software, Erithacus Software, Horley, UK).

### Results

Binding affinities at the ORL-1 receptor and cytochrome P450 inhibitions are reported in Table 1

**Table 1**

| **Example No.** | **ORL-1 Binding affinity** | **CYP450 interaction** | | | | |
|---|---|---|---|---|---|---|
| | | **(IC₅₀, uM)** | | | | |
| | **(Kᵢ, nM)** | **1A2** | **2C9** | **2C19** | **2D6** | **3A4** |
| 7 | 4.8 | >100 | 20.2 | 25.1 | 16.8 | 13.3 |
| 6 | 34 | >100 | >100 | >100 | 5.1 | 98.2 |
| 3 | 4.2 | >100 | 25.9 | 47.5 | 8.6 | 54.7 |
| 12 | 2.8 | >100 | >100 | >100 | 36.6 | 18.2 |
| 13 | 18 | >100 | 98.6 | 62.6 | 94.6 | 38.1 |
| 15 | 8.2 | >100 | 74.4 | 38.3 | 34.7 | 6.2 |
| 11 | 5.5 | >100 | >100 | 47.4 | 20.7 | 9.6 |

As evident, the present compounds showed a marked binding affinity vs. the ORL-1 receptor, joined with a very low inhibiting activity of P450.

The P450 inhibitory activity of the present invention was compared with that of WO2005005411, example 53. The results are presented in the Table 2.

As evident, the compounds of the invention were found to have P450 inhibiting activity from 3 to 1000 times lower than the reference compound.

**Table 2**

| **Ex. No** | **Structure** | **CYP450 interaction** | | | | |
|---|---|---|---|---|---|---|
| | | **(IC₅₀, uM)** | | | | |
| | | **1A2** | **2C9** | **2C19** | **2D6** | **3A4** |
| 53 (W02005005411) | 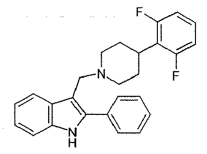 | 11.8 | 10.7 | 17.4 | <0.1 | 13.0 |
| 13 | 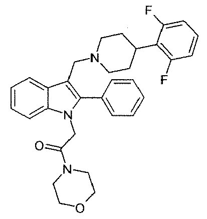 | >100 | 98.6 | 62.6 | 94.6 | 38.1 |
| 12 | 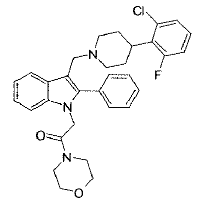 | >100 | >100 | >100 | 36.6 | 18.2 |
| 15 | 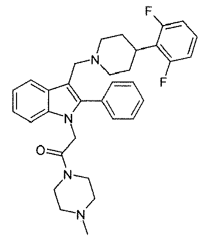 | >100 | 74.4 | 38.3 | 34.7 | 6.2 |
| 8 | 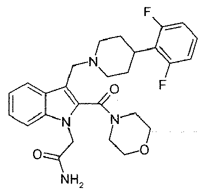 | 86.0 | 31.2 | 70.1 | 6.2 | 25.6 |
| 6 | 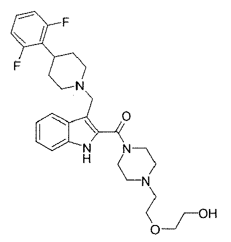 | >100 | >100 | >100 | 5.1 | 98.2 |

## Claims

1. Compound of formula (I) 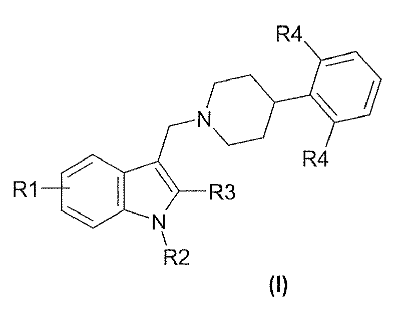 or a salt or hydrate thereof, wherein:
**R1** is hydrogen; halogen; C₁₋₆alkyl; perhaloC₁₋₆alkyl; aryl;
**R2** and **R3** are, independently from each other:
hydrogen; C₁₋₆alkyl, aryl; arylC₁₋₆alkyl; -(CH₂)ₙCONRₐR_{b} where n = 0-4; Rₐ and R_{b} are, independently from each other: hydrogen; C₁₋₆alkyl; C₃₋₆cycloalkyl; aryl; arylC_{1- 6}alkyl; heteroarylC₁₋₆alkyl; saturated or unsaturated, optionally substituted heterocycle containing 1 to 3 heteroatoms chosen from O, S, N; or Rₐ and R_{b} together with the nitrogen atom to which they are attached, form a saturated or unsaturated, optionally substituted heterocycle containing a total of 1 to 3 heteroatoms chosen from O, S, N, and whose ring members may be bound with each other by a C₁₋₄ alkyl bridge, or said heterocycle may be spiro-substituted at any position by an optionally substituted nitrogen-containing heterocycle, and with the proviso that at least one among R2 and R3 is -(CH₂)ₙCONRₐR_{b};
the **R4** groups are, independently from each other, hydrogen or halogen, with the proviso that at least one R4 group is a fluorine atom.

2. Compound according to claim 1 where, in the -(CH₂)ₙCONRₐR_{b} group, n is 0 or 1; Rₐ and R_{b} are hydrogen or an optionally substituted saturated heterocycle containing 1 to 3 heteroatom chosen from O, S, N, or Rₐ and R_{b} together with the nitrogen atom to which they are attached, form an optionally substituted saturated heterocycle containing 1 to 3 heteroatoms chosen from O, S, N, whose ring members may be bound with each other by a C₁₋₂alkyl bridge.

3. Compound according to claims 1-2, where the -(CH₂)ₙCONRₐR_{b} group is chosen from -CONH₂, -CH₂-CONH₂, or one of the following structures: 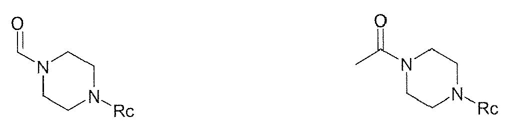 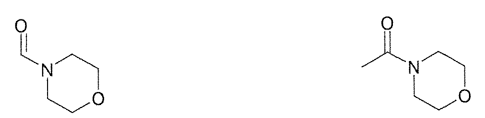 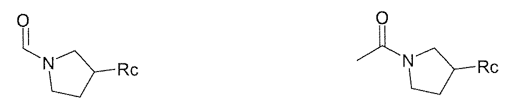 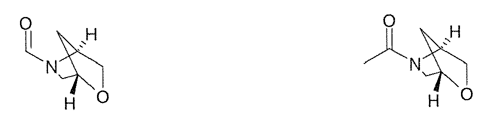 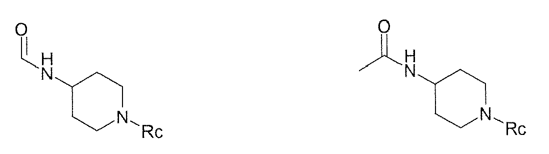 where R_{c} is C₁₋₆ linear or branched alkyl, C₁₋₆alkylCON(R_{d}) where R_{d} is H or C₁₋₆ linear or branched alkyl, hydroxyC₁₋₆ alkyl wherein one member of said alkyl can be replaced by oxygen, heterocyclylcarbonyl or heterocyclylcarbonylC₁₋₆alkyl.

4. Compound according to claims 1-3, where said heterocyclyl present in R_{c} is chosen from piperidin-1-yl, morpholin-4-yl, piperazin-1-yl.

5. Compound according to claims 1-4, where R1 is chosen among hydrogen or halogen.

6. Compound according to claim 1, chosen from:
2-(4-{3-[4-(2-Chloro-6-fluoro-phenyl)-piperidin-1-ylmethyl]-1H-indole-2-carbonyl}-piperazin-1-yl)-1-morpholin-4-yl-ethanone
2-(4-{3-[4-(2,6-Difluoro-phenyl)-piperidin-1-ylmethyl]-1H-indole-2-carbonyl}-piperazin-1-yl)-1-morpholin-4-yl-ethanone
N-(1-{3-[4-(2-Chloro-6-fluoro-phenyl)-piperidin-1-ylmethyl]-1H-indole-2-carbonyl}-pyrrolidin-3-yl)-N-methyl-acetamide
N-(1-{3-[4-(2,6-Difluoro-phenyl)-piperidin-1-ylmethyl]-1H-indole-2-carbonyl}-pyrrolidin-3-yl)-N-methyl-acetamide
{3-[4-(2-Chloro-6-fluoro-phenyl)-piperidin-1-ylmethyl]-1H-indol-2-yl}-{4-[2-(2-hydroxy-ethoxy)-ethyl]-piperazin-1-yl}-methanone
{3-[4-(2,6-Difluoro-phenyl)-piperidin-1-ylmethyl]-1H-indol-2-yl}-{4-[2-(2-hydroxyethoxy)-ethyl]-piperazin-1-yl}-methanone
2-[3-[4-(2-Chloro-6-fluoro-phenyl)-piperidin-1-ylmethyl]-2-(morpholine-4-carbonyl)-indol-1-yl]-acetamide
2-[3-[4-(2,6-Difluoro-phenyl)-piperidin-1-ylmethyl]-2-(morpholine-4-carbonyl)-indol-1-yl]-acetamide
{3-[4-(2-Chloro-6-fluoro-phenyl)-piperidin-1-ylmethyl]-1H-indol-2-yl}-(1S,4S)-2-oxa-5-aza-bicyclo[2.2.1]hept-5-yl-methanone
{3-[4-(2,6-Difluoro-phenyl)-piperidin-1-ylmethyl]-1H-indol-2-yl}-(1S,4S)-2-oxa-5-aza-bicyclo[2.2.1]hept-5-yl-methanone hydrochloride
2-{3-[4-(2-Chloro-6-fluoro-phenyl)-piperidin-1-ylmethyl]-2-methyl-indol-1-yl}-N-(1-methyl-piperidin-4-yl)-acetamide
2-{3-[4-(2-Chloro-6-fluoro-phenyl)-piperidin-1-ylmethyl]-2-phenyl-indol-1-yl}-1-morpholin-4-yl-ethanone
2-{3-[4-(2,6-Difluoro-phenyl)-piperidin-1-ylmethyl]-2-phenyl-indol-1-yl}-1-morpholin-4-yl-ethanone
2-{3-[4-(2-Chloro-6-fluoro-phenyl)-piperidin-1-ylmethyl]-2-phenyl-indol-1-yl}-1-(4-methy)-piperazin-1-yl)-ethanone
2-{3-[4-(2,6-Difluoro-phenyl)-piperidin-1-ylmethyl]-2-phenyl-indol-1-yl}-1-(4-methyl-piperazin-1-yl)-ethanone
2-{3-[4-(2,6-Difluoro-phenyl)-piperidin-1-ylmethyl]-2-methyl-indol-1-yl}-1-(4-methyl-piperazin-1-yl)-ethanane

7. Compound according to claims 1-6, being isotopically labelled.

8. Process for preparing the compounds of formula (I), as defined in claim 1, **characterised by** binding together, in any order, the following building blocks: 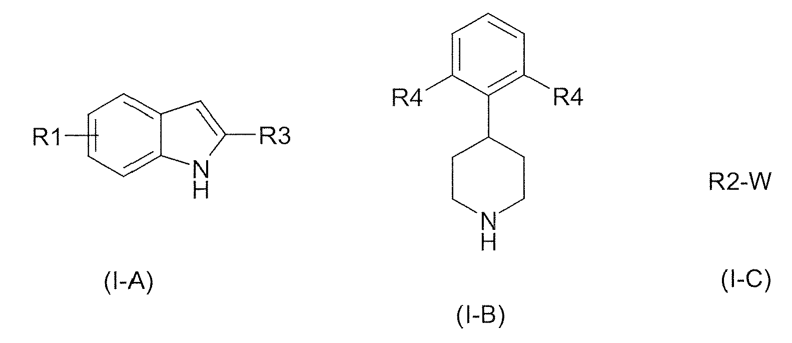 where R1-R4 are as defined in claim 1, where at least one among R2 and R3 is the group -(CH₂)ₙCONRₐR_{b} as defined in claim 1 or a synthetic precursor thereof, and where W is a suitable leaving group.

9. Process according to claim 8, where block (I-B) is added to block (I-A) in presence of formaldehyde and an acid, in the conditions of Mannich reaction.

10. Process according to claims 8-9, where block (I-C) is added to the NH group of block (I-A) by mean of nucleophilic substitution, in basic conditions.

11. Process according to claims 8-10, wherein said precursor ia a group of formula -(CH₂)ₙCOOH, or -(CH₂)ₙCOOR where n is as defined in claim 1 and R is a linear or branched C1-4 alkyl group.

12. Process according to claim 11, wherein said precursor is converted into the - (CH₂)ₙCONRₐR_{b} by reaction of said group -(CH₂)ₙCOOR with a suitable activated amine of formula NHRₐR_{b}, or by reaction of said group -(CH₂)ₙCOOH, suitably activated, with an amine of formula NHRₐR_{b}.

13. Process according to claim 12, wherein said activation is obtained by: (i) contacting said amine HNRₐR_{b} with a suitable activating agent such as trimethylaluminium, or (ii) converting said COOH group into the corresponding acyl halide.

14. Compound of formula (I) as defined in claim 1, for use in therapy.

15. Pharmaceutical compositon comprising an active agent of formula (I) as defined in claim 1, in association with one or more pharmaceutical excipients.

16. Pharmaceutical composition with reduced risk of unwanted drug-drug interactions containing in addition to said compound of formula (I) as defined in claim 1, a further active agent to be administered in co-therapy therewith.

17. Use of a compound of formula (I) as defined in claim 1, in the manufacture of a medicament for the prevention and/or treatment of diseases dependent on the activation of the ORL-1 receptor.

18. Use according to claim 17, where said disease is selected from pain conditions and Parkinson's disease.

19. Use of a compound of formula (I) as defined in claim 1, in the manufacture of a medicament for antagonising the activity of the ORL-1 receptor in a mammal in need thereof.
